Europäisches Patentamt

European Patent Office

Office européen des brevets

(19)

(11) Veröffentlichungsnummer: **0 127 607**
**A1**

## (12) EUROPÄISCHE PATENTANMELDUNG

(21) Anmeldenummer: **84890091.6**

(22) Anmeldetag: **18.05.84**

(51) Int. Cl.³: **A 61 N 5/00**

(30) Priorität: **18.05.83 AT 1827/83**

(43) Veröffentlichungstag der Anmeldung:
**05.12.84 Patentblatt 84/49**

(84) Benannte Vertragsstaaten:
**BE CH DE FR GB IT LI LU NL SE**

(71) Anmelder: **Robek, Franc**
**Vinska gora 24**
**YU-63320 Titovo Velenje(YU)**

(72) Erfinder: **Robek, Franc**
**Vinska gora 24**
**YU-63320 Titovo Velenje(YU)**

(74) Vertreter: **Piso, Eberhard, Dr.**
**Patentanwälte Dipl.-Ing. Herbert C.E. Krause Dr.**
**Eberhard Piso Gluckgasse 1**
**A-1010 Wien 1(AT)**

(54) **Elektrophysikalisches Therapiegerät.**

(57) Elektrophysikalisches Therapiegerät mit mindestens einem HF-Oszillator und einer Stromquelle, wobei die einem Parallelschwingkreis (11, 16) zugeordneten Drahtwindungen (23, 28) des HF-Oszillators mindestens einen zentralen Aktivator (17, 22) umschließen, der über einen Draht (29) mit einem Außenkontakt (9) in Wirkverbindung steht.

Fig.1

EP 0 127 607 A1

Elektrophysikalisches Therapiegerät

Die Erfindung betrifft ein elektrophysikalisches Therapiegerät mit mindestens einem HF-Oszillator und einer Stromquelle.

Bekanntlich ist Leben, technisch definiert, ein ständiger Ablauf höchst komplexer elektrochemischer Prozesse, und jede Veränderung eines einzelnen Faktors in den zahllosen Regelkreisen, die das "Funktionieren" des menschlichen Körpers gewährleisten, zieht unausweichlich Veränderungen vieler anderer Faktoren nach sich.

Bisher wurden einem Menschen, dessen Wohlbefinden beeinträchtigt ist bzw. bei dem sich Krankheitssymptome zeigten, in der Regel Chemotherapeutika verabreicht, d.h. es wurde versucht, die ablaufenden Prozesse vornehmlich von der chemischen Seite her zu beeinflussen. So wird z.B. zur Regelung von Magen- und Körper- bzw. Harnsäure sowie des Blutzuckerspiegels oder des Blutdrucks auf Normalwerte einem unter zu hohen oder zu niedrigen Werten leitenden Patienten häufig eine medikamentöse Behandlung verordnet. Gleiches gilt auch für das allgemeine Wohlbefinden, eine Steigerung der Vitalität und die Erhöhung der körperlichen Widerstandskraft. Da aber die meisten Medikamente unerwünschte Nebenwirkungen zeigen, vor allem starke Präparate bzw. bei Langzeitmedikation, ist deren Einnahme nicht ungefährlich und kann zu weiteren krankhaften Neben- bzw. Folgeerscheinungen führen, die die Behandlung des Patienten wesentlich verteuern sowie seine Leistungsfähigkeit zumindest zeitweise stark mindern. Außerdem muß der behandelnde Arzt genau unterscheiden, ob er einen neurotischen bzw. hypochondrischen oder einen körperlich kranken Menschen zu behandeln hat, da bei einem körperlich gesunden Menschen ein Medikament, das dieser wegen eines eingebildeten Krankheitszustandes einnimmt, möglicherweise gerade erst Schädigungen des Körpers hervorrufen kann.

Anderseits wurde aber auch versucht, Krankheitssymptome bzw. Erkrankungen durch Diät oder durch Änderungen des Energiezustandes des Körpers bzw. von Körperteilen beispielsweise durch Bestrahlung, Kältezufuhr, Massage, Bäder zu beeinflussen, um krankhafte Zustände zu bessern.

Ebenso ist anzunehmen, daß Akupunktur die elektrischen Potentiale im menschlichen Körper beeinflußt.

Aufgabe der Erfindung ist es, ein Gerät zu schaffen, das mit gleichbleibender Intensität Fehlfunktionen im lebenden Organismus ausgleicht und eine Heilung krankhafter Zustände sowie Hebung des Wohlbefindens und Steigerung der Vitalität des Organismus ohne gefährliche Nebenwirkungen auf einfache und billige Weise hervorruft und sichert.

Die Erfindung löst diese Aufgabe dadurch, daß in den Drahtwindungen eines Parallelschwingkreises mindestens ein zentraler Aktivator als Parallelinduktivität eingesetzt ist, der über einen Draht mit einem Außenkontakt in Wirkverbindung steht. Über diesen Außenkontakt kann an den menschlichen Körper eine vom Aktivator abhängige komplexe Energieform abgegeben werden.

Eine weitere Ausgestaltung der Erfindung liegt darin, daß der zentrale Aktivator ein geschlossener, mit einer Aktivatorsubstanz gefüllter Behälter ist, daß der mit dem Außenkontakt in Wirkverbindung stehende Draht in den Behälter des zentralen Aktivators hineinreicht und zumindest zum Teil von der Aktivatorsubstanz umgeben ist. Eine solche Ausführungsform gewährleistet, daß die Aktivatorsubstanz von Umwelteinflüssen unbehelligt bleibt und die Abgabe der gewünschten Energie an den Benützer des Gerätes gewährleistet ist.

Erfindungsgemäß ist vorgesehen, daß der Behälter eine Glasampulle, vorzugsweise aus K-Na-Glas ist, oder daß der Behälter aus Keramik besteht, wobei die Drahtwindungen des Parallelschwingkreises in der Wandung des Behälters des zentralen Aktivators angeordnet sein können.

Die Erfindung besteht auch darin, daß die Aktivatorsubstanz aus einem Pulver eines oder mehrerer Metalle oder Halbmetalle, deren Oxiden und/oder Salzen besteht, daß die Metalle und Halbmettalle ausgewählt sind aus Ge, Tm, Na, K, Tb, Fe, Al, Ca, Mg, Si, P und daß die Salze Chloride und/oder Fluoride sind.

Ferner besteht die Erfindung auch darin, daß der im Aktivator angeordnete, mit dem Außenkontakt in Wirkverbindung stehende Draht ein oberflächenoxidierter Fe-Draht ist, daß der zentrale Aktivator von jeweils fünf Drahtwindungen des Parallelschwingkreises umgeben ist, daß die Resonanzfrequenz zur Erregung der Aktivatorsubstanz im Bereich von 80 bis 300 MHz liegt und daß im Parallelschwingkreis mehrere, von diesem einzeln einschaltbare Parallelinduktivitäten angeordnet sind.

Weiters besteht die Erfindung auch darin, daß jedem Parallelschwingkreis mit einer Parallelinduktivität ein Oszillator zugeordnet ist. Wenn der zentrale Aktivator von einem Hochfrequenzfeld durchflossen wird, gibt er eine in Abhängigkeit von seinem Inhalt modifizierte Energieform an den Außenkontakt und von dort an den Benutzer des Therapiegerätes ab. Bei Anordnung von mehreren Aktivatoren ist erfindungsgemäß vorgesehen, daß jeweils immer nur ein Aktivator einschaltbar ist und daß alle zentralen Aktivatoren mit dem Außenkontakt in ständiger Verbindung sind.

Außerdem ist die Erfindung auch dadurch gekennzeichnet, daß der Draht des Parallelschwingkreises ein oberflächenoxidierter Fe-Draht ist. Die Intensität der an den Benutzer abgegebenen Energie ist proportional der Frequenz. Erfindungsgemäß ist auch vorgesehen, daß der mit dem Außenkontakt in Verbindung stehende Draht unterbrechbar ist.

Das erfindungsgemäße elektrotherapeutische Gerät wurde in langjähriger experimenteller Arbeit im Bereich der Radiästesie entwickelt. Überraschend ist, daß die vom Gerät abgestrahlte Energieform, die vermutlich (ohne Festlegung auf diese Theorie ) vom Erregungszustand der Aktivatorsubstanz(en) abhängt, je nach Inhalt der zentralen Aktivatoren auf den lebenden Organismus die gewünschte heilende bzw. vitalitätssteigernde Wirkung ausübt. Wie zahlreiche Versuche inzwischen gezeigt haben, können mit dem erfindungsgemäßen Gerät vor allem Störungen im Bereich der Magen- und Körpersäure (Harnsäure), des Blutzuckers und des Blutdrucks erfolgreich behandelt werden, wobei abhängig von dem gewählten Aktivator bzw. der Einstellung des Gerätes eine Erhöhung bzw. Senkung der betreffenden Werte erfolgt. Ebenso wurden Entzündungen und Reizzustände gelindert.

Durch die Handlichkeit des erfindungsgemäßen Therapiegeräts, seine kleinen Abmessungen, sein geringes Gewicht, ergibt sich der Vorteil, daß es gewünschtenfalls ständig mitgetragen werden kann und, soferne es eingeschaltet ist, auch ständig wirkt. Der Patient ist nicht nur nicht mehr gezwungen, genau und zeitgerecht die für ihn bestimmten Medikamente einzunehmen, sondern er braucht auch keine unerwünschten Nebenerscheinungen, wie sie mit vielen Medikamenten, z.B. den Cortisonpräparaten, einhergehen, zu fürchten. Es besteht weder die Gefahr der Gewöhnung (Sucht) noch die der Ansammlung giftiger Substanzen, die bei Einnahme starker Medikamente bzw. bei Dauermedikation auch eine psychische zusätzliche Belastung des Patienten bedeuten kann. Abgesehen davon, kann eine Behandlung, aus welchen Gründen auch immer, durch Abschaltung des Gerätes sofort abgebrochen werden. Weiters sind jene Schwierigkeiten, die das Schlucken großer Kapseln oder Tabletten manchen Patienten verursacht, aus dem Weg geräumt.

Ein weiterer Vorteil des erfindungsgemäßen Geräts besteht auch darin, daß es beispielsweise ein hypochondrisch veranlagter Mensch benutzen kann, ohne daß es negative Auswirkungen auf seinen Gesundheitszustand hätte, da die vom Gerät erzeugte Energieform die Vitalität steigert und so dem körperlichen und dadurch auch dem geistigen Wohlbefinden dient und der Regenerierung sowie einer Steigerung der Widerstandskraft des Organismus förderlich ist.

Die Erfindung ist in der Zeichnung an Hand von Ausführungsbeispielen näher veranschaulicht.

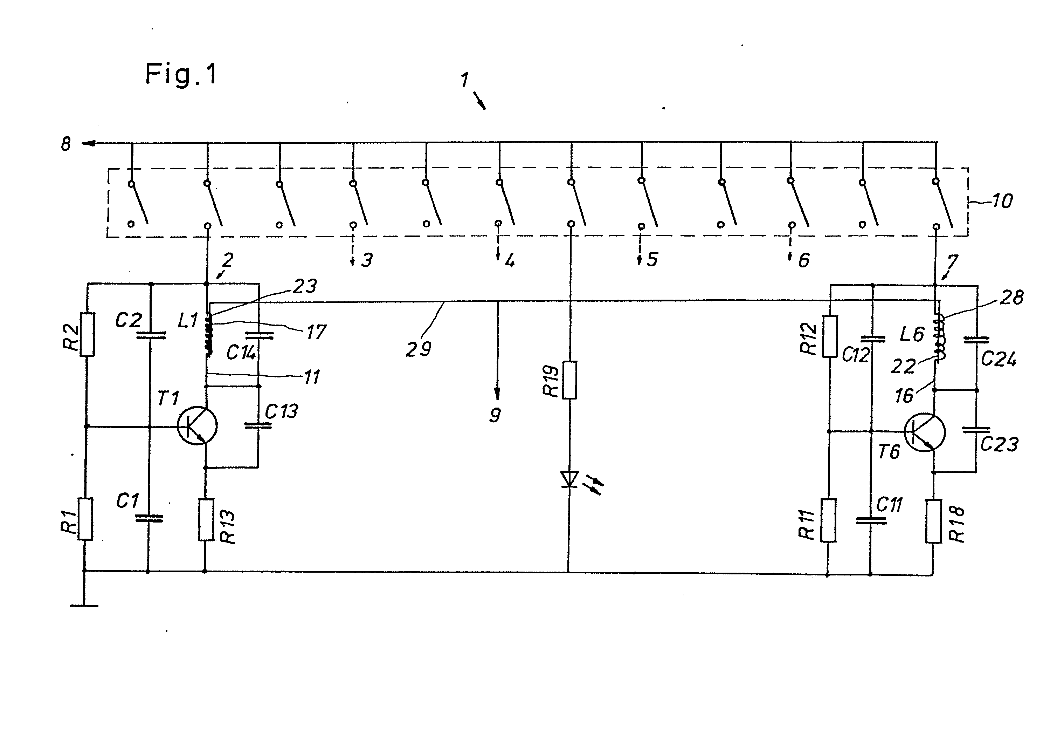

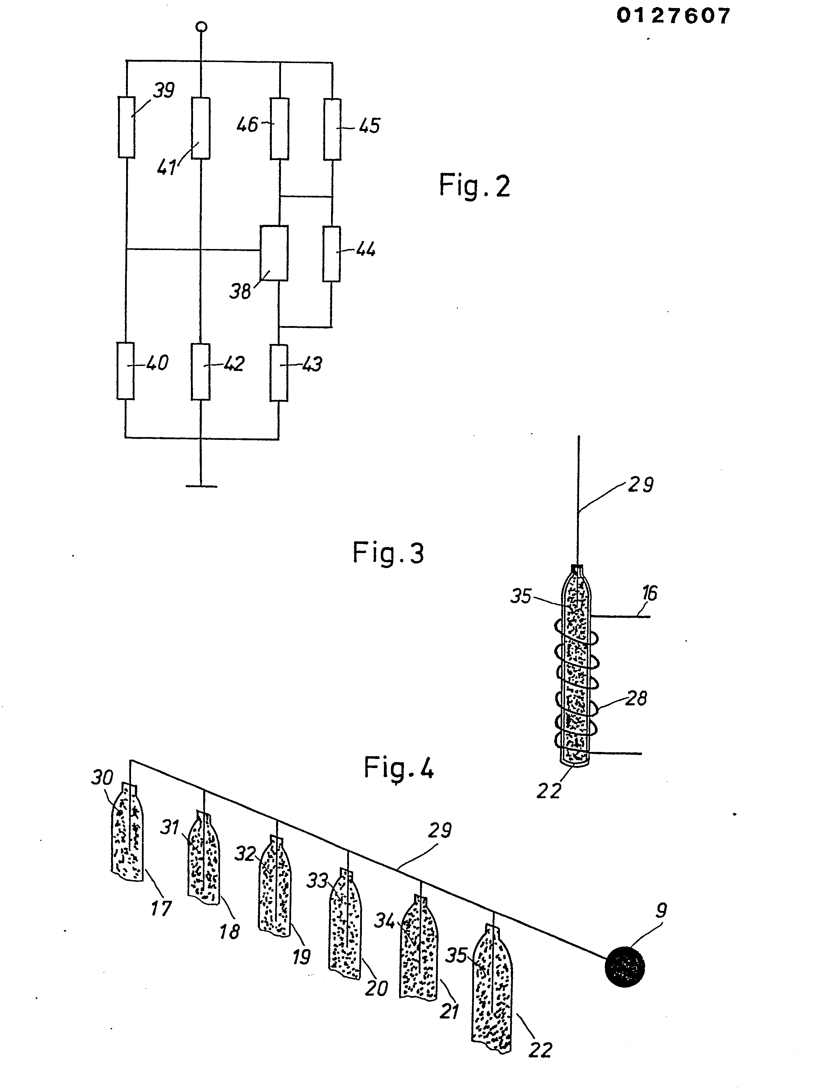

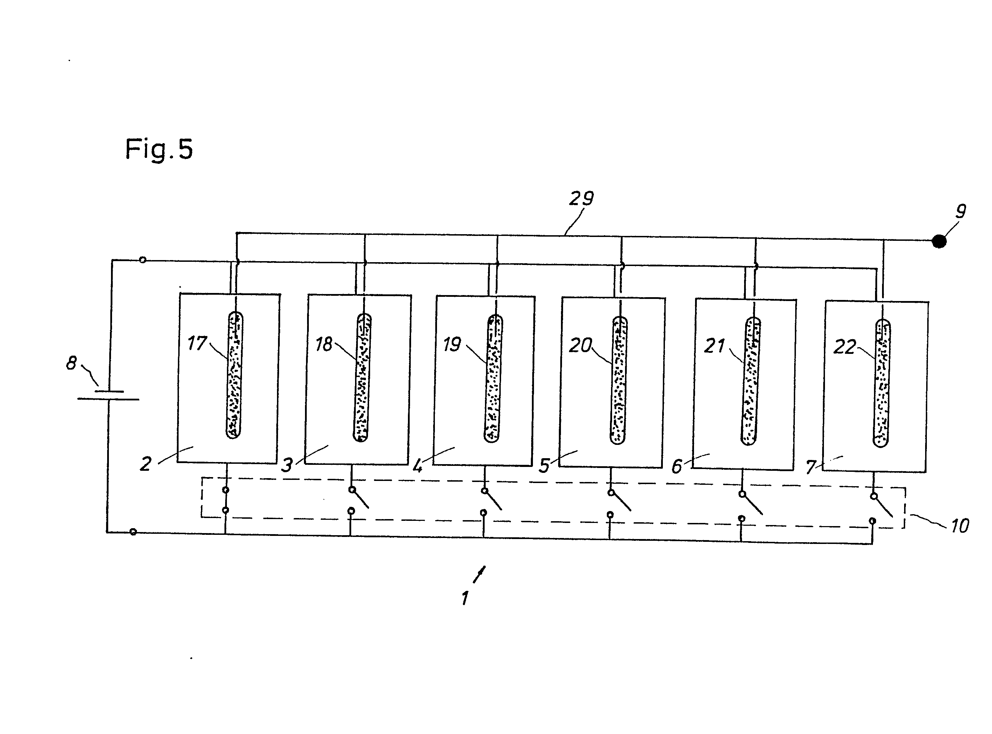

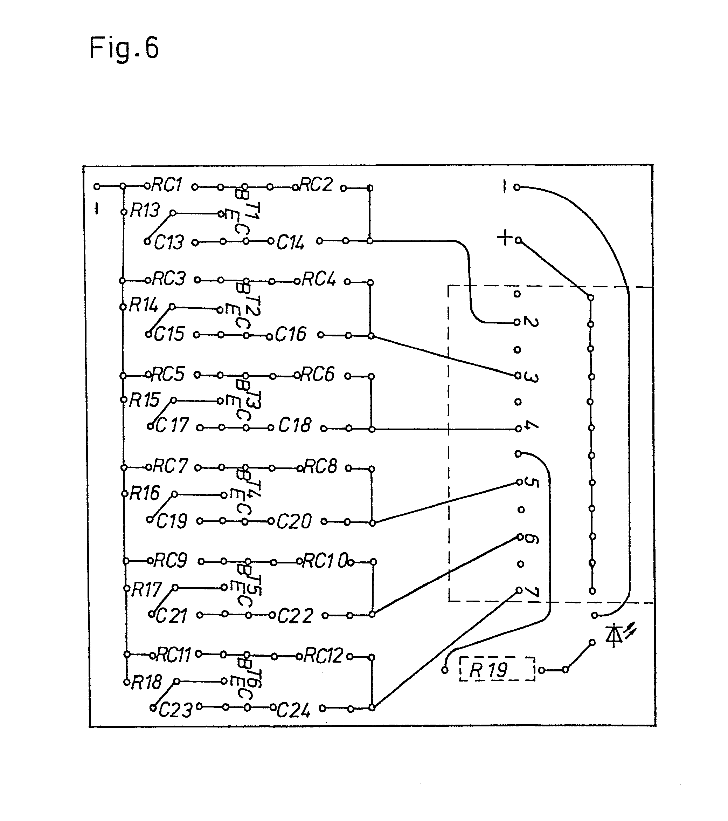

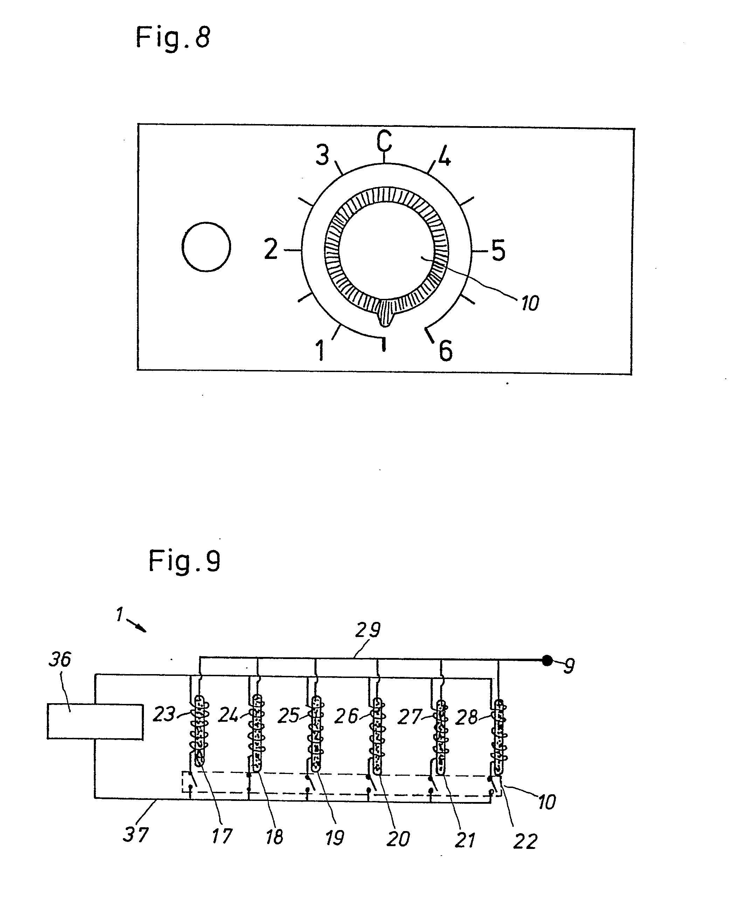

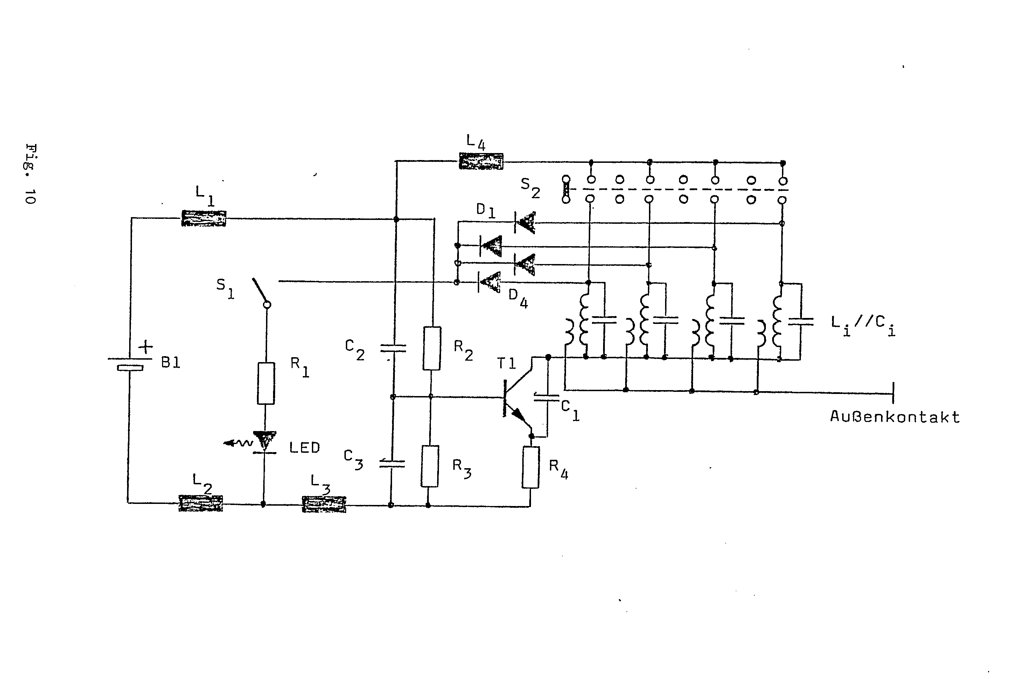

Es zeigen die Fig. 1 einen Oszillatorenkreis mit festen Parallelinduktivitäten, Fig. 2 einen einzelnen Oszillator als Blockschaltbild, Fig. 3 einen zentralen Aktivator, Fig. 4 die Zusammenschaltung mehrerer zentraler Aktivatoren mit dem Außenkontakt und die Fig. 5 ein Blockschaltbild des Oszillatorenkreises aus Fig. 1. Die Fig. 6 und 7 zeigen die Ausführung der gedruckten Schaltung des Oszillatorenkreises, Fig. 8 die Gerätebedienung und die Fig. 9 eine andere Variante des Erfindungsgegenstandes mit auswechselbaren Parallelinduktivitäten. Fig. 10 zeigt den elektronischen Stromlaufplan einer Ausführungsform des erfindungsgemäßen Therapiergerätes, an dem Messungen ausgeführt wurden.

Aus der Zeichnung ist zu ersehen, daß über einen mehrpoligen Funktionswahlschalter 10, der als Dreh- aber auch als Schiebeschalter mit markierter Raststellung ausgebildet sein kann, ein selektives Einschalten der Hochfrequenzoszillatoren 2 bis 7 bzw. 36 bzw. der Kontaktschluß z.B. einer Spule 23 des Parallelschwingkreises 11 mit dem Oszillator 2 erfolgt, wobei die Spule mit 1 bis 10, vorzugsweise jedoch 5 Windungen die ihr zugeordnete Parallelinduktivität 17 umschließt. Der bzw. die Oszillatoren 2 bis 7 bzw. 36 stehen mit einer nach bestimmten Kriterien sorgsam ausgewählten Batterie 8 in Wirkverbindung. Eine Auswahl der Batterie 8 soll deshalb erfolgen, da diese, was ihre chemische Zusammensetzung (Anode, Kathode und Elektrolyt) anbelangt, einen gewissen Einfluß auf die Wirkungsweise des Therapiegerätes haben kann. Nach Herstellung einer Verbindung des jeweiligen Parallelschwingkreises 11 bis 16 bzw. 37 und Stromfluß durch dessen Spule 23 bis 28 wird um eine Ampulle 17 bis 22 von etwa 4 bis 10 mm Durchmesser und etwa 40 mm Länge, die aus K-Na-Glas, aber auch aus Porzellan sein kann, ein Hochfrequenzfeld aufgebaut. Dies erfolgt von einem modifizierten Hochfrequenzgenerator mit einer Resonanzfrequenz von ca. 80 MHz bis etwa 300 MHz und mehr. Die Bestimmung der Resonanzfrequenz erfolgt durch den jeweiligen Parallelschwingkreis 11 bis 16 bzw. 37, wobei sie jedoch für die Funktion des Gerätes 1 relativ unerheblich ist. Die Intensität der vom Gerät abgestrahlten Energieform ist proportional der Frequenz. Nieder- und mittelfrequente Ströme, die die Spule 23 bis 28 um die Ampulle 17 bis 22 durchströmen, haben die gleiche Funktion wie hochfrequente Ströme. Nach erfolgtem Aufbau des Feldes beispielsweise des Parallelschwingkreises 11 des Oszillators 2 wird die in der Ampulle 17 befindliche Substanz 30 in einen Erregungszustand versetzt, und über einen oberflächenoxidierten Eisendraht 29, der auch die restlichen, in Spulen 23 bis 28 eingebundenen zentralen Aktivatoren 18 bis 22 ständig miteinander verbindet, wird eine komplexe Energieform an den Außenkontakt 9 des Gerätes 1 abgestrahlt.An und für sich reicht es sogar aus, wenn der Patient das erfindungsgemäße Therapiegerät 1 bei sich trägt, z.B. in der Brusttasche, wird jedoch die Haut vom Kontakt 9 berührt, so geht der Energiefluß zur Gänze in den Organismus über, wo er den jeweils erwünschten Effekt, z.B. Heilung bestimmter Leiden, Steigerung des Wohlbefindens, Regulierung des Blutdrucks, usw. durch Einsatz des jeweiligen Aktivators 17 bis 22 bewirkt.

Die für den Eisendraht der Spulen 23 bis 28 der Parallelschwingkreise 11 bis 16 bzw. 37 und der Verbindung 29 zwischen den zentralen Aktivatoren 17 bis 22 und dem Außenkontakt 9 verwendeten Stärken entsprechen etwa 0,1 bis 1 mm, wobei das Gerät 1 jedoch auch mit anderen Durchmessern, auch der Ampullen 17 bis 22, funktioniert, wobei die hier angegebenen Werte eine Optimierung des Gerätes 1 in seiner Wirkung auf den lebenden Organismus bedeuten.

Die in die Spulenkörper 17 bis 22 gefüllten Substanzen 30 bis 35 sind von zentraler Bedeutung und können vor allem aus vierzehn ausgewählten chemischen Elementen und deren Verbindungen bestehen, die bei Erregung durch das Hochfrequenzfeld energetisch zusammenwirken, in der Ampulle 14 bis 22 aber keiner Akkumulation ausgesetzt sind. Diese Elemente sind beispielsweise:

| Periode/Gruppe | Element | Gewichtsprozente | | |
|---|---|---|---|---|
| | | von | - | bis |
| 4 / IV | Ge, Germanium | 0,3 | - | 0,6 % |
| Lat. | Tm, Tulium | 0,2 | - | 1,3 % |
| 3 / I | Na, Natrium | 24 | - | 23 % |
| 4 / I | K, Kalium | 10 | - | 13 % |
| Lat. | Tb, Terbium | 0,1 | - | 0,2 % |
| 4 / VIII | Fe, Eisen | 29,7 | - | 27,3 % |
| 3 / III | Al, Aluminium | 34 | - | 32 % |
| 4 / II | Ca, Calcium | 1 | - | 1 % |
| 3 / II | Mg, Magnesium | 0,2 | - | 0,2 % |
| 3 / IV | Si, Silicium | 0,3 | - | 0,8 % |
| 3 / V | P, Phosphor | 0,2 | - | 0,4 % |
| 2 / VI | O, Sauerstoff | in den Verbindungen | | |
| 3 / VII | Cl, Chlor | in den Verbindungen | | |
| 2 / VII | Fl, Fluor | in den Verbindungen | | |

Zur Einflußnahme auf Magensäure und Blutzucker werden z.B. vorzugsweise folgende Elemente in der Ampulle verwendet: Aluminium, Tulium, Natrium, Kalium, Silicium, Germanium.

Der Parallelschwingkreis des Oszillators besteht aus einem Kondensator, z.B. C 14 von 2 pF - 6,8 pF und der als zentraler Aktivator bezeichneten Parallelinduktivität, z.B. 11 mit Spule 23, wobei das Gerät meist mit einer Kombination von 1 bis 6, aber auch mehr Aktivatoren 17 bis 22 ausgestattet ist, die jedoch vorzugsweise immer nur einzeln und nicht in Wirkungskombinationen eingeschaltet werden. Die Lebensdauer der zentralen Aktivatoren 17 bis 22, d.h. ihres Inhalts 30 bis 35, ist theoretisch unbegrenzt, da ihr Abbau, der durch das Hochfrequenzfeld des Parallelschwingkreises 11 bis 16 bzw. 37 erfolgt, Jahrzehnte in Anspruch nimmt und immer nur sehr geringe Mengen Energie, auch bei Dauerbetrieb, an die nächste Umgebung des Gerätes abgegeben werden.

Der Aufbau der Hochfrequenzoszillatoren 2 bis 7, aber auch des Oszillators 36 der in Fig. 9 gezeigten, Bauteile einsparenden Variante ist wie folgt:

Teil 38 ist ein HF-Transistor in Basisschaltung, Teil 39 und Teil 40 bilden den Basisspannungsteiler, Teil 41 und Teil 42 sind die erforderlichen Kapazitäten für einen Oszillatorbetrieb in Basisschaltung. Teil 43 ist der erforderliche Emitterwiderstand zur Ruhestromeinstellung und Teil 44 ist die für eine Oszillation notwendige Rückkopplungskapazität. Der Teil 45 bildet die Parallelkapazität und Teil 46 die Parallelinduktivität 17 bis 22 des Parallelschwingkreises 11 bis 16 bzw. 37.

Die Parallelinduktivität 17 bis 22 ist, wie bereits geschildert, eine Glasampulle, gefüllt mit ausgewählten Elementen bzw. deren Verbindungen 30 bis 35. Sie bildet den Spulenkörper für die aus vorzugsweise 5 Windungen Eisendraht bestehende Spule 23 bis 28. Die Ampulle 17 bis 22 ist mit einer Eisendrahtzuführung 29 verschweißt.

Die Eisendrahtzuführungen 29 aller Spulenkörper 17 bis 22, auch zentrale Aktivatoren genannt, sind alle gemeinsam zu einem Eisenkontakt 9 am Geräteäußeren geführt und an diesem, der dem direkten Hautkontakt dient, angeordnet.

Der in der Fig. 9 gezeigte Geräteaufbau ist im Funktionsprinzip gleich der Variante der Fig. 1, jedoch kommt nur ein Oszillator 36 mit seinem Parallelschwingkreis 37 zum Einsatz, in dem die vorgesehenen Parallelinduktivitäten 17 bis 22 angeordnet sind und je nach Belieben in den Prozeß mittels des Funktionswahlschalters 10 einzeln eingeschaltet werden können.

Es sei besonders hervorgehoben, daß die Erfindung weder auf die dargestellten Ausführungsformen, noch auf die erwähnten chemischen Elemente und deren Verbindungen oder Mischungsverhältnisse beschränkt ist. Ebenso kann die Anzahl der die Aktivatoren umgebenden Drahtwindungen innerhalb des Gerätes variieren.

Ein Ausführungsbeispiel des erfindungsgemäßen Therapiegerätes mit dem in Fig. 10 dargestellten elektronischen Stromlaufplan wurde auf seine elektronische Funktionstüchtigkeit hin untersucht. Die Batterie B 1 war eine herkömmliche 9 V Trockenbatterie, wie sie vorzüglich in portablen Geräten wie Taschenradios, Rechnern usw. Verwendung findet. Die Induktivitäten $L_1$ bis $L_4$ sind als Filter zu bezeichnende Eisendrähte unterschiedlicher Länge und Formgebung, deren Induktivitätsmessung nicht möglich war.

Die aktive Komponente des in Basisschaltung arbeitenden Oszillators war ein Hf-Transistor T 1 der Type BFW 92, wie er von der Firma Siemens hergestellt wird. Der Basisspannungsteiler $R_2$ (100k), $R_3$ (47k) war so gewählt, daß er unter Normalbedingungen ohne Berücksichtigung des Basisstromes einen Arbeitspunkt bei $U_B$ = 2,8 V festlegt. Die beiden Kondensatoren $C_2$ und $C_3$ (10nF) dienen lediglich zur Entkopplung.

Der Emitterwiderstand $R_4$ (1k) ist so gewählt, daß eine hohe Schwingneigung des Oszillators gegeben ist, was für die im Kollektorkreis

angeordneten, durch Umschalter $S_2$ zu wählenden Parallelschwingkreise geringer elektronischer Güte (bedingt durch oxidierte Eisendrahtwindungen und stark absorbierende Kerne) notwendig war. Diese Parallelschwingkreise mit ihren Spulenkernen (Glasampullen mit diversen Salzen und einer Auskopplungwicklung aus oxidiertem Eisendraht) waren dabei Hauptgegenstand der Untersuchung.

Der Kondensator $C_1$ ist die Rückkopplung für die Verstärkerstufe mit T 1. Die Funktionskontrolle $S_1$ mit LED, $R_1$ (330) und Dioden $D_1$, $D_2$, $D_3$, $D_4$ (1N4148) sind hilfreiches Accessoire für den Benützer, tragen aber zur Wirkung des Gerätes nichts bei.

Die Intensität der Oszillationen konnte von der Verschiebung des Arbeitspunktes abgelesen werden, so ergab sich für:

| Schalterstellung | $C_i$ | $L_i$ | $V_B$ | $\Delta I_{rel}$* |
|---|---|---|---|---|
| 1 | $C_{11}$ | $L_{11}$ | 1,963V | -2,41 |
| 2 | $C_{12}$ | $L_{12}$ | 1,965V | -1,40 |
| 5 | $C_{14}$ | $L_{14}$ | 1,971V | 1,65 |
| 6 | $C_{13}$ | $L_{13}$ | 1,972V | 2,16 |

*$\Delta I_{rel}$ ist auf den Mittelwert aller Intensitäten bezogen und in Promille angegeben.

Im Hinblick auf die Frequenzen der Oszillationen muß man eigentlich im vorliegenden Fall von der Grundfrequenz der Schwingung sprechen; sie lagen allesamt etwas über 200 MHz und waren neben der Abhängigkeit von der Batteriespannung und den Temperaturkoeffizienten der verwendeten Bauteile noch von dem Umfeld des Gerätes (Berührung mit der Hand usw.) sehr stark abhängig.

Die Kurvenform der Oszillationen wich geringfügig von der idealen Sinusschwingung ab, was sicherlich mit den verwendeten Spulenkernen (Salze von Seltenerdmetallen usw.) in engem Zusammenhang steht. Eine genaue Quantifizierung war mit den üblichen Mitteln nicht möglich.

Zusammenfassend wurde bei diesem Versuch festgestellt, daß es sich bei dem geprüften erfindungsgemäßen Therapiegerät um einen Generator handelt, der in vier Stufen unterschiedliche Hochfrequenzschwingungen komplexer Formgebung ausstrahlt.

Patentansprüche :

1. Elektrophysikalisches Therapiegerät mit mindestens einem HF-Oszillator und einer Stromquelle, dadurch gekennzeichnet, daß die einem Parallelschwingkreis (11 bis 16 bzw. 17) zugeordneten Drahtwindungen (23 bis 28) des HF-Oszillators mindestens einen zentralen Aktivator (17 bis 22) umschließen, der über einen Draht (29) mit einem Außenkontakt (9) in Wirkverbindung steht.

2. Elektrophysikalisches Therapiegerät nach Anspruch 1, dadurch gekennzeichnet, daß der zentrale Aktivator (17 bis 22) ein geschlossener, mit einer Aktivatorsubstanz (30 bis 35) gefüllter Behälter ist.

3. Elektrophysikalisches Therapiegerät nach Anspruch 1 oder 2, dadurch gekennzeichnet, daß der mit dem Außenkontakt (9) in Wirkverbindung stehende Draht (29) in den Behälter des zentralen Aktivators (17 bis 22) hineinreicht und zumindest zum Teil von der Aktivatorsubstanz (30 bis 35) umgeben ist.

4. Elektrophysikalisches Therapiegerät nach einem der Ansprüche 1 bis 3, dadurch gekennzeichnet, daß der Behälter (17 bis 22) eine Glasampulle, vorzugsweise aus K-Na-Glas ist.

5. Elektrophysikalisches Therapiegerät nach einem der Ansprüche 1 bis 3, dadurch gekennzeichnet, daß der Behälter (17 bis 22) aus Porzellan ist.

6. Elektrophysikalisches Therapiegerät nach einem der Ansprüche 1 bis 5, dadurch gekennzeichnet, daß die Drahtwindungen (23 bis 28) in der Wandung des Behälters des zentralen Aktivators (17 bis 22) angeordnet sind.

7. Elektrophysikalisches Therapiegerät nach einem der Ansprüche 1 bis 6, dadurch gekennzeichnet, daß die Aktivatorsubstanz (30 bis 35) aus einem Pulver einer oder mehrerer Metalle oder Halbmetalle, deren Oxiden und/oder Salzen besteht.

8. Elektrophysikalisches Therapiegerät nach Anspruch 7, dadurch gekennzeichnet, daß die Metalle und Halbmetalle ausgewählt sind aus Ge, Tm, Na, K, Tb, Fe, Al, Ca, Mg, Si, P.

9. Elektrophysikalisches Therapiegerät nach Anspruch 7, dadurch gekennzeichnet, daß die Salze Chloride und/oder Fluoride sind.

10. Elektrophysikalisches Therapiegerät nach einem der Ansprüche 1 bis 9, dadurch gekennzeichnet, daß der im Aktivator (17 bis 22) angeordnete, mit dem Außenkontakt (9) in Wirkverbindung stehende Draht (29) oberflächenoxidierter Fe-Draht ist.

11. Elektrophysikalisches Therapiegerät nach einem der Ansprüche 1 bis 10, dadurch gekennzeichnet, daß der zentrale Aktivator (17 bis 22) von jeweils fünf Drahtwindungen (23 bis 28) des Parallelschwingkreises (11 bis 16 bzw. 37) umgeben ist.

12. Elektrophysikalisches Therapiegerät nach einem der Ansprüche 1 bis 11, dadurch gekennzeichnet, daß die Resonanzfrequenz zur Erregung der Aktivatorsubstanz (30 bis 35) im Bereich von 80 bis 300 MHz liegt.

13. Elektrophysikalisches Therapiegerät nach einem der Ansprüche 1 bis 12, dadurch gekennzeichnet, daß im Parallelschwingkreis (11 bis 16 bzw. 37) mehrere, von diesem einzeln wegschaltbare zentrale Aktivatoren (17 bis 22) angeordnet sind.

14. Elektrophysikalisches Therapiegerät nach einem der Ansprüche 1 bis 12, dadurch gekennzeichnet, daß jedem Parallelschwingkreis (11 bis 16 bzw. 37) mit einem zentralen Aktivator (17 bis 22) ein Oszillator (2 bis 7 bzw. 36) zugeordnet ist.

15. Elektrophysikalisches Therapiegerät nach einem der Ansprüche 1 bis 14, dadurch gekennzeichnet, daß bei Anordnung von mehreren Aktivatoren (17 bis 22) jeweils immer nur ein Aktivator einschaltbar ist.

16. Elektrophysikalisches Therapiegerät nach einem der Ansprüche 1 bis 15, dadurch gekennzeichnet, daß alle zentralen Aktivatoren (17 bis 22) mit dem Außenkontakt (9) in ständiger Verbindung sind.

17. Elektrophysikalisches Therapiegerät nach einem der Ansprüche 1 bis 16, dadurch gekennzeichnet, daß der Draht des Parallelschwingkreises (11 bis 16 bzw. 37) ein oberflächenoxidierter Fe-Draht ist.

18. Elektrophysikalisches Therapiegerät nach einem der Ansprüche 1 bis 17, dadurch gekennzeichnet, daß der mit dem Außenkontakt (9) in Verbindung stehende Draht (29) unterbrechbar ist.

Fig.1

0127607

0127607

Fig.2

Fig.3

Fig.4

0127607

Fig.5

Fig.6

0127607

Fig.8

Fig.9

Fig. 10

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | FR-A-1 347 133 (SCHENK)<br>* Seite 1, die drie ersten Abschnitte und vorletzter Abschnitt * | 1-4,7 | A 61 N 5/00 |
| A | DE-A-3 013 601 (PRACHT)<br>* Seiten 2,3 * | 1,2,4 | |
| A | FR-A-1 571 268 (LEBEDEFF)<br><br>* Seite 1, die zwei letzten Abschnitte * | 1,10, 17 | |
| A | DE-A-3 114 905 (RASCHE)<br>* Seite 13; Seite 14, erster Abschnitt; Seite 17, letzter Abschnitt; Seite 19, Abschnitt 1 * | 1-3,13 | |
| A | FR-A-1 604 700 (SCHNEIDER)<br>* Seite 1, Zeilen 25-33; Seite 2, Zeilen 5-8 * | 1-4,16 | RECHERCHIERTE SACHGEBIETE (Int. Cl. 3)<br><br>A 61 N |
| A | DE-C- 841 779 (MATZKE)<br>* Seite 2, Zeilen 100-113 * | 2,4 | |

Der vorliegende Recherchenbericht wurde für alle Patentansprüche erstellt.

| Recherchenort<br>DEN HAAG | Abschlußdatum der Recherche<br>23-07-1984 | Prüfer<br>SIMON J.J.E. |
|---|---|---|